# FASCICULE DE BREVET EUROPEEN

(11) **EP 3 391 006 B1**
(45) Date de publication et mention de la délivrance du brevet: **02.12.2020**
(21) Numéro de dépôt: 16813103.5
(22) Date de dépôt: 07.12.2016
(51) Int. Cl.: G01M 3/04, A61N 5/06

(54) **DISPOSITIF DE DETECTION D'UNE FUITE DANS UNE ENCEINTE HERMETIQUE**
SYSTEM ZUR ERKENNUNG EINES LECKS IN EINEM VERSIEGELTEN GEHÄUSE
DEVICE FOR DETECTING A LEAK IN A SEALED ENCLOSURE

(30) Priorité: 17.12.2015 FR 1562697
(43) Date de publication de la demande: 24.10.2018
(73) Titulaire: Commissariat à l'Energie Atomique et aux Energies Alternatives, 75015 Paris (FR)
(72) Inventeur: CHABROL, Claude, 38320 Poisat (FR); LAURENT, Jean-Yves, 38420 Domene (FR); ROUTIN, Julien, 38500 La Buisse (FR)
(74) Mandataire: Cabinet Beaumont
(86) Numéro de dépôt international: PCT/FR2016/053257
(87) Numéro de publication internationale: WO 2017/103382

(56) Documents cités:
- WO-A1-2008/082362
- US-A1- 2013 194 199
- A. P. GHOSH ET AL: "Thin-film encapsulation of organic light-emitting devices", APPLIED PHYSICS LETTERS, vol. 86, no. 22, 1 janvier 2005 (2005-01-01), page 223503, XP055006694, ISSN: 0003-6951, DOI: 10.1063/1.1929867
- SMITH JOSEPH T ET AL: "Application of Flexible OLED Display Technology for Electro-Optical Stimulation and/or Silencing of Neural Activity", JOURNAL OF DISPLAY TECHNOLOGY, IEEE SERVICE CENTER, NEW YORK, NY, US, vol. 10, no. 6, 1 juin 2014 (2014-06-01), pages 514-520, XP011549012, ISSN: 1551-319X, DOI: 10.1109/JDT.2014.2308436 [extrait le 2014-05-20]

## Description

La présente demande de brevet revendique la priorité de la demande de brevet français FR15/62697.

### Domaine

La présente demande concerne un dispositif de détection d'une fuite dans une enceinte hermétique. La présente demande concerne en particulier la détection d'une fuite dans une enceinte hermétique destinée à être implantée dans le corps humain ou animal.

### Exposé de l'art antérieur

Il existe un besoin croissant pour des dispositifs électroniques implantables dans le corps humain ou animal, par exemple pour la mise en oeuvre de stimulations électriques ou optiques d'organes internes du corps humain ou animal. Ces dispositifs peuvent notamment comporter des circuits électroniques intégrant des matériaux non biocompatibles. Dans ce cas, le dispositif peut comporter une enceinte ou un boîtier biocompatible encapsulant de façon hermétique les composants non biocompatibles. L'herméticité du boîtier permet notamment d'éviter que du liquide physiologique n'entre en contact avec les composants non biocompatibles du dispositif, ce qui pourrait provoquer une intoxication du patient. L'herméticité du boîtier permet en outre de limiter les risques de corrosion des composants du dispositif.

Pour améliorer la sécurité du patient, il serait souhaitable de pouvoir détecter in-situ, c'est-à-dire sans avoir à retirer le dispositif du corps du patient, une éventuelle fuite dans une enceinte hermétique d'un dispositif médical implantable.

Diverses méthodes ont été proposées pour détecter une fuite dans un boîtier hermétique. Ces méthodes sont toutefois relativement complexes, et, pour certaines, ne peuvent être mises en oeuvre qu'à l'extérieur du corps du patient. En outre, les méthodes connues sont mal adaptées à la détection de fuite dans des enceintes de petit volume, par exemple de volume inférieur à 10 mm³, que l'on peut rencontrer dans le domaine des dispositifs médicaux implantables.

Le document WO2008082362 décrit un exemple de dispositif encapsulé intégrant un capteur de perméabilité au gaz. L'article intitulé "Thin-film encapsulation of organic light-emitting devices" de A. P. Gosh et al (Applied Physics Letters, vol. 86, no. 22, 1 janvier 205) présente une étude sur des couches d'encapsulation de diodes électroluminescentes organiques. Le document US2013/194199 décrit un dispositif d'affichage à base de diodes électroluminescentes organiques, comportant des photodiodes.

### Résumé

Ainsi, un mode de réalisation prévoit un dispositif de détection d'une fuite dans une enceinte hermétique, comportant une diode électroluminescente organique destinée à être placée à l'intérieur de l'enceinte ; et un dispositif de mesure d'une grandeur représentative d'au moins l'un des paramètres suivants : a) le rendement lumineux de la diode ; et b) l'impédance de la diode.

Selon un mode de réalisation, le dispositif comprend en outre un circuit de traitement adapté à comparer ladite grandeur à une ou plusieurs valeurs de référence, et à en déduire la présence éventuelle d'oxygène ou d'humidité dans des proportions anormales à l'intérieur de l'enceinte.

Selon un mode de réalisation, le circuit de traitement est adapté à commander une alarme.

Selon un mode de réalisation, le dispositif de mesure comprend un photodétecteur placé en vis-à-vis de la diode électroluminescente organique.

Selon un mode de réalisation, le photodétecteur est réalisé à base d'un matériau semiconducteur inorganique.

Un autre mode de réalisation prévoit un dispositif médical comportant une enceinte hermétique biocompatible, et un dispositif de détection d'une fuite dans l'enceinte hermétique tel que défini ci-dessus.

Selon un mode de réalisation, l'enceinte hermétique est transparente et contient une source de stimulation optique du cerveau.

Selon un mode de réalisation, l'enceinte hermétique comprend un tube transparent.

Selon un mode de réalisation, le tube est fermé hermétiquement à ses extrémités par des bouchons.

Selon un mode de réalisation, le dispositif comporte des éléments de connexion électrique traversant au moins l'un des bouchons et reliant électriquement à l'extérieur de l'enceinte des composants situés à l'intérieur du tube.

### Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :
la figure 1 représente schématiquement un exemple d'un mode de réalisation d'un dispositif de détection de la présence d'une fuite dans une enceinte hermétique ;
la figure 2 représente schématiquement une variante de réalisation du dispositif de la figure 1 ; et
la figure 3 illustre schématiquement un exemple d'un mode de réalisation d'un dispositif médical comportant une enceinte hermétique biocompatible et un dispositif de détection de la présence d'une fuite dans cette enceinte.

### Description détaillée

De mêmes éléments ont été désignés par de mêmes références aux différentes figures et, de plus, les diverses figures ne sont pas tracées à l'échelle. Par souci de clarté, seuls les éléments qui sont utiles à la compréhension des modes de réalisation décrits ont été représentés et sont détaillés. En particulier, les divers composants (autres que les composants du dispositif de détection de fuite) susceptibles d'être disposés dans un boîtier biocompatible hermétique en vue d'une implantation dans le corps d'un patient n'ont pas été représentés et ne sont pas détaillés, les modes de réalisation décrits étant compatibles avec tous les dispositifs implantables connus comportant un boîtier d'encapsulation hermétique. Sauf précision contraire, les expressions "approximativement", "sensiblement", et "de l'ordre de" signifient à 10 % près, de préférence à 5 % près.

Selon un aspect d'un mode de réalisation, on prévoit d'utiliser une diode électroluminescente organique (OLED) disposée à l'intérieur d'une enceinte hermétique pour détecter la présence éventuelle d'une fuite dans cette enceinte.

Il est connu que les diodes électroluminescentes organiques (OLED) voient leurs performances se dégrader en présence d'humidité ou d'oxygène. En particulier, en présence d'humidité ou d'oxygène, le rendement lumineux d'une diode électroluminescente organique décroit rapidement, et son impédance augmente rapidement.

Selon un aspect d'un mode de réalisation, on prévoit d'exploiter ce défaut connu des diodes électroluminescentes organiques pour détecter une présence d'humidité ou d'oxygène dans des proportions anormales dans une enceinte hermétique, et en déduire la présence possible d'une fuite dans l'enceinte.

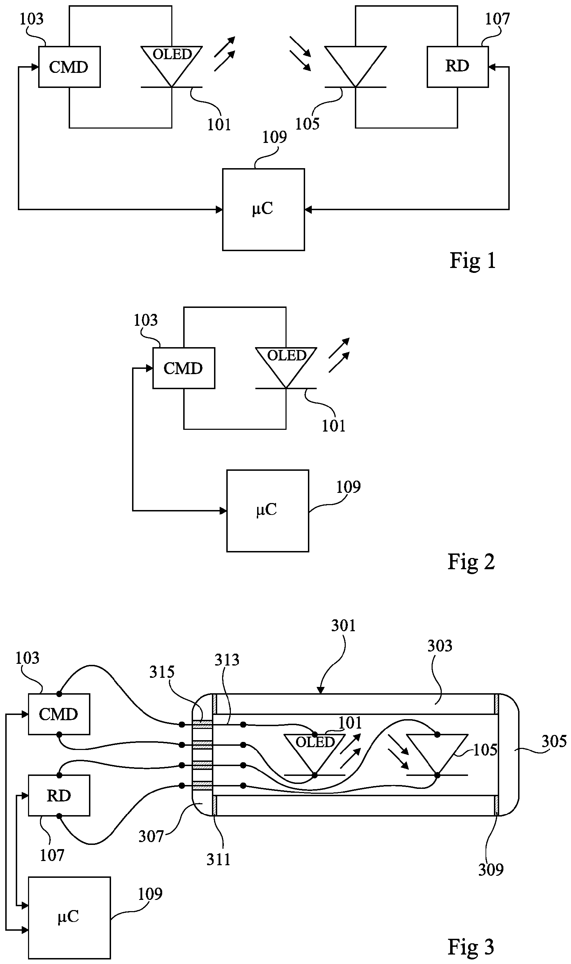

La figure 1 est une représentation schématique d'un exemple d'un mode de réalisation d'un dispositif de détection d'une fuite dans une enceinte hermétique (non représentée).

Le dispositif de la figure 1 comporte une diode électroluminescente organique 101 (OLED) destinée à être placée à l'intérieur de l'enceinte hermétique. La diode 101 comprend au moins une couche en un matériau semiconducteur organique disposée entre une électrode d'anode et une électrode de cathode de la diode. Lorsqu'un courant adapté est appliqué entre l'anode et la cathode de la diode 101, des photons sont générés par la couche semiconductrice organique, et la diode 101 émet de la lumière. On notera qu'il existe de nombreuses technologies de réalisation de diodes électroluminescentes organiques, basées sur l'utilisation de matériaux semiconducteurs organiques différents, et/ou sur des agencements différents de ces matériaux. Toutefois, toutes les diodes électroluminescentes organiques connues ont pour point commun d'être particulièrement sensibles à l'humidité et/ou à l'oxygène. Ainsi, les modes de réalisation décrits peuvent être mis en oeuvre quel que soit le type de la diode électroluminescente organique 101 utilisée.

Le dispositif de la figure 1 comprend en outre un module ou circuit 103 d'alimentation et de commande (CMD) de la diode 101, connecté à l'anode et à la cathode de la diode 101. Le module 103 comprend par exemple une batterie électrique ou toute autre source d'alimentation adaptée, ainsi qu'un circuit permettant de commander la diode 101 à l'état allumé ou éteint.

Le dispositif de la figure 1 comprend de plus un photodétecteur 105 disposé en regard de la diode 101, adapté à fournir un signal électrique représentatif de l'intensité lumineuse émise par la diode 101. Le photodétecteur 105 est par exemple une photodiode. Le photodétecteur 105 peut être réalisé à base d'un matériau semiconducteur inorganique, par exemple du silicium.

Le dispositif de la figure 1 comprend par ailleurs un module ou circuit 107 de lecture (RD) d'une grandeur électrique représentative du niveau d'éclairement reçu par le photodétecteur 105.

De plus, le dispositif de la figure 1 comprend un circuit de traitement 109 (µC), comportant par exemple un microprocesseur, ce circuit étant relié au module de lecture 107 et pouvant en outre être relié au module d'alimentation et de commande 103.

Le fonctionnement du dispositif de la figure 1 est le suivant. Lors d'une phase de détection, le dispositif 103 commande l'allumage de la diode 101. Pour cela, le dispositif 103 applique par exemple à la diode 101 une tension d'alimentation fixe prédéterminée, ou un courant d'alimentation fixe prédéterminé, ou une puissance (courant*tension) d'alimentation fixe prédéterminée. Lorsque la diode 101 est allumée, l'ensemble formé par le photodétecteur 105 et le module de lecture 107 mesure une grandeur représentative de l'intensité lumineuse émise par la diode 101. A partir de cette mesure, et connaissant la puissance d'alimentation électrique fournie à la diode 101, le circuit de traitement 109 peut estimer le rendement lumineux de la diode 101. Le circuit de traitement 109 compare le rendement mesuré à une ou plusieurs valeurs de référence, et peut ainsi détecter une diminution anormale du rendement lumineux de la diode 101, et en déduire la présence d'humidité ou d'oxygène dans des proportions anormales à l'intérieur de l'enceinte. En particulier, si le rendement lumineux de la diode 101 est plus faible qu'attendu, le circuit de traitement 109 peut en déduire que le taux d'oxygène et/ou le taux d'humidité à l'intérieur de l'enceinte est anormalement élevé, ce qui a conduit à dégrader prématurément le rendement de la diode. Le circuit de traitement 109 peut notamment en déduire la présence d'une fuite dans l'enceinte hermétique contenant la diode 101. Lorsque le circuit de traitement 109 détecte une fuite dans l'enceinte, il déclenche par exemple une alarme pour signaler cette fuite.

Pour déterminer les valeurs de référence de rendement lumineux utilisées par le circuit de traitement 109 pour détecter la présence éventuelle d'une fuite dans l'enceinte, une phase de caractérisation de la diode 101 peut être prévue à la conception du dispositif. En particulier, l'évolution en fonction du temps du rendement lumineux de la diode 101 peut être déterminée d'une part pour une utilisation normale du dispositif, c'est-à-dire en l'absence de fuite dans l'enceinte hermétique contenant la diode 101, et d'autre part en cas de fuite. Les lois de variation du rendement lumineux déterminées lors de la phase de caractérisation peuvent être mémorisées dans le circuit de traitement 109, par exemple sous forme de tables.

La figure 2 est une représentation schématique d'une variante de réalisation du dispositif de détection de fuite de la figure 1.

Le dispositif de la figure 2 diffère du dispositif de la figure 1 principalement en ce qu'il ne comprend pas le photodétecteur 105 et le module de lecture 107 du dispositif de la figure 1.

Dans le dispositif de la figure 2, le module 103 d'alimentation et de commande (CMD) de la diode 101 est adapté à mesurer l'impédance de la diode 101. Pour cela, le module 103 est par exemple adapté à appliquer une tension prédéterminée entre l'anode et la cathode de la diode 101, et à mesurer le courant circulant dans la diode 101 pendant que cette tension est appliquée. A titre de variante, le module 103 est adapté à appliquer un courant prédéterminé entre l'anode et la cathode de la diode 101, et à mesurer la tension aux bornes de la diode 101 pendant que ce courant est appliqué. Le module d'alimentation et de commande 103 de la diode 101 est adapté à communiquer au circuit de traitement 109 les valeurs d'impédance mesurées.

Le fonctionnement du dispositif de la figure 2 est le suivant. Lors d'une phase de détection, le dispositif 103 mesure l'impédance de la diode 101, par exemple à l'état allumé (c'est-à-dire que la tension et le courant appliqués à la diode 101 lors de la mesure d'impédance sont adaptés à provoquer l'allumage de la diode 101). Le circuit 109 compare la valeur d'impédance mesurée à une ou plusieurs valeurs de référence, et peut ainsi détecter une augmentation anormale de l'impédance de la diode 101, et en déduire la présence d'humidité ou d'oxygène dans des proportions anormales à l'intérieur de l'enceinte. Le circuit de traitement 109 peut notamment en déduire la présence d'une fuite dans l'enceinte hermétique contenant la diode 101, et, le cas échéant, déclencher une alarme pour signaler cette fuite.

Pour déterminer les valeurs de référence d'impédance utilisée par le circuit 109 pour détecter la présence éventuelle d'une fuite dans l'enceinte, une phase de caractérisation de la diode 101 peut être prévue à la conception du dispositif. En particulier, l'évolution en fonction du temps de l'impédance de la diode 101 peut être déterminée d'une part pour une utilisation normale du dispositif, c'est-à-dire en l'absence de fuite dans l'enceinte hermétique contenant la diode 101, et d'autre part en cas de fuite. Les lois de variation de l'impédance déterminées lors de la phase de caractérisation peuvent être mémorisées dans le circuit de traitement 109, par exemple sous forme de tables.

On notera que les variantes de réalisation décrites en relation avec les figures 1 et 2 peuvent être combinées, c'est-à-dire que le dispositif de détection de fuite peut exploiter à la fois des mesures représentatives du rendement lumineux de la diode 101 et des mesures représentatives de l'impédance de la diode 101 pour détecter une éventuelle variation du taux d'humidité ou du taux d'oxygène dans l'enceinte hermétique contenant la diode 101, et en déduire la présence d'une fuite dans cette enceinte.

La figure 3 est une vue en coupe représentant de façon schématique et partielle un exemple d'un mode de réalisation d'un dispositif médical comportant une enceinte hermétique biocompatible implantable 301 et un dispositif du type décrit en relation avec les figures 1 et 2 permettant de détecter la présence éventuelle d'une fuite dans l'enceinte 301.

Le dispositif de la figure 3 est un dispositif de stimulation optique d'organes internes du corps humain ou animal, par exemple un dispositif de stimulation optique profonde du cerveau. L'enceinte 301 contient une source lumineuse de stimulation (non représentée), et est destinée à être implantée à l'intérieur du cerveau, en vis-à-vis d'une partie du cerveau que l'on souhaite stimuler.

L'enceinte 301 comprend un tube 303 en un matériau biocompatible transparent, par exemple du saphir ou de la silice, à l'intérieur duquel est disposée la source lumineuse de stimulation. Le tube 303 a par exemple une section circulaire. A titre d'exemple, la largeur du tube 303 (c'est-à-dire son diamètre dans le cas d'un tube à section circulaire) est comprise entre 0,5 et 5 mm, et sa longueur est comprise entre 1 et 10 mm. L'enceinte 301 comprend en outre des bouchons 305 et 307 fermant hermétiquement le tube 303 à ses extrémités. Les bouchons 305 et 307 sont par exemple transparents. A titre d'exemple, les bouchons 305 et 307 sont en le même matériau biocompatible transparent que le tube 303. Le bouchon 305 est soudé sur toute sa périphérie à une première extrémité du tube 303 par une brasure hermétique biocompatible 309, par exemple une brasure à l'or. Le bouchon 307 est soudé sur toute sa périphérie à l'extrémité opposée du tube 303 par une brasure hermétique biocompatible 311, par exemple une brasure à l'or. La fixation des bouchons 305 et 307 aux extrémités du tube 303 est par exemple réalisée par soudure thermosonique ou par thermocompression. Une fois les bouchons 305 et 307 en place, l'ensemble comprenant le tube 303 et les bouchons 305 et 307 forme un boîtier hermétique adapté à isoler l'ensemble des composants non biocompatibles qu'il contient, par exemple un boîtier présentant un taux de fuite à l'hélium inférieur à 10⁻⁶ atm.cm³/s et de préférence inférieur à 10⁻⁸ atm.cm³/s, avec 1 atm = 101325 Pa. A titre d'exemple, le boîtier 109 est fermé sous atmosphère neutre (sans oxygène), par exemple sous azote ou argon.

Le dispositif de la figure 3 comprend en outre un dispositif de détection de fuite du type décrit en relation avec la figure 1. La diode électroluminescente organique 101 du dispositif de détection de fuite est disposée à l'intérieur de l'enceinte 301. Le photodétecteur 105 du dispositif de détection de fuite est également disposé à l'intérieur de l'enceinte, en vis-à-vis de la diode 101. Dans cet exemple, le module 103 d'alimentation et de commande de la diode 101, le module 107 de lecture du photodétecteur 105 et le circuit de traitement 109 du dispositif de détection de fuite sont disposés à l'extérieur de l'enceinte 301. A titre d'exemple, les éléments 103, 107 et 109 peuvent être disposés à l'extérieur du corps du patient, ou être implantés dans une autre partie du corps du patient (à l'extérieur du cerveau).

Dans l'exemple représenté, des éléments de connexion électrique 313 traversant l'enceinte 301 relient électriquement à l'extérieur de l'enceinte les composants situés dans l'enceinte, et en particulier la diode 101 et le photodétecteur 105. Dans l'exemple représenté, chaque élément de connexion électrique 313 comprend une tige conductrice traversant de part en part le bouchon 307 par l'intermédiaire d'un trou ou via percé dans ce bouchon. Chacun des vias est fermé hermétiquement par une brasure 315, par exemple une brasure à l'or. Un câble de liaison peut être prévu pour relier électriquement les éléments de connexion électrique 313 aux modules 103 et 107.

Plus généralement, les dispositifs de détection de fuite décrits peuvent équiper tout type de boîtier hermétique biocompatible (transparent ou non) destiné à être implanté dans le corps d'un patient.

Un avantage des dispositifs de détection de fuite décrits est qu'ils sont particulièrement simples à mettre en oeuvre. De plus, ces dispositifs sont adaptés à la détection de fuite dans des enceintes de petit volume. Par ailleurs, on notera que la diode électroluminescente organique 101 peut être utilisée non seulement pour la surveillance d'étanchéité de l'enceinte dans laquelle elle est disposée, mais aussi pour d'autres fonctions, par exemple comme émetteur de lumière pour l'irradiation optique du cerveau du patient dans le cas d'un dispositif de stimulation optique du type décrit en relation avec la figure 3.

Des modes de réalisation particuliers ont été décrits. Diverses variantes et modifications apparaîtront à l'homme de l'art. En particulier, les modes de réalisation décrits ne se limitent pas à la détection de fuite dans des boîtiers hermétiques biocompatibles implantables, mais peuvent plus généralement avoir d'autres applications, notamment hors du domaine médical.

## Revendications

1. Dispositif comportant :
une enceinte hermétique ;
une diode électroluminescente organique (101) placée à l'intérieur de l'enceinte ;
un dispositif (103, 105, 107) de mesure d'une grandeur représentative d'au moins l'un des paramètres suivants :
a) le rendement lumineux de la diode (101) ; et
b) l'impédance de la diode (101) ; et
un circuit de traitement (109) mémorisant, dans l'alternative a), une loi représentative de l'évolution en fonction du temps du rendement lumineux de la diode (101) en l'absence de fuite dans l'enceinte hermétique et une loi représentative de l'évolution en fonction du temps du rendement lumineux de la diode (101) en présence d'une fuite dans l'enceinte hermétique, et, dans l'alternative b), une loi représentative de l'évolution en fonction du temps de l'impédance de la diode (101) en l'absence de fuite dans l'enceinte hermétique et une loi représentative de l'évolution en fonction du temps de l'impédance de la diode (101) en présence d'une fuite dans l'enceinte hermétique, lesdites lois étant déterminées lors d'une phase de caractérisation à la conception du dispositif, et le circuit de traitement étant adapté à comparer ladite grandeur à une ou plusieurs valeurs de référence issues desdites lois, et à en déduire la présence éventuelle d'oxygène ou d'humidité dans des proportions anormales à l'intérieur de l'enceinte,
dans lequel le circuit de traitement (109) est adapté à commander une alarme lorsqu'il détecte une présence d'oxygène ou d'humidité dans des proportions anormales à l'intérieur de l'enceinte.

2. Dispositif selon la revendication 1, dans lequel le dispositif de mesure comprend un photodétecteur (105) placé en vis-à-vis de la diode électroluminescente organique (101).

3. Dispositif selon la revendication 2, dans lequel le photodétecteur (105) est réalisé à base d'un matériau semiconducteur inorganique.

4. Dispositif médical comportant une enceinte hermétique (301) biocompatible, et un dispositif selon l'une quelconque des revendications 1 à 3 de détection d'une fuite dans l'enceinte hermétique (301).

5. Dispositif médical selon la revendication 4, dans lequel l'enceinte hermétique (301) est transparente, et contient une source de stimulation optique du cerveau.

6. Dispositif médical selon la revendication 5, dans lequel l'enceinte hermétique (301) comprend un tube transparent (303) .

7. Dispositif médical selon la revendication 6, dans lequel le tube (303) est fermé hermétiquement à ses extrémités par des bouchons (305, 307).

8. Dispositif selon la revendication 7, comportant des éléments de connexion électrique (313) traversant au moins l'un des bouchons (305, 307) et reliant électriquement à l'extérieur de l'enceinte (301) des composants situés à l'intérieur du tube (303) .

## Patentansprüche

1. Eine Vorrichtung, die Folgendes aufweist:
ein abgedichtetes Gehäuse;
eine organische lichtemittierende Diode (101), die innerhalb des Gehäuses angeordnet ist;
eine Vorrichtung (103, 105, 107) zum Messen einer Größe, die für wenigstens einen der folgenden Parameter repräsentativ ist:
a) der Lichtausbeute der Diode (101); und
b) der Impedanz der Diode (101); und
eine Verarbeitungsschaltung (109), die Folgendes speichert, in der Alternative a) ein Gesetz, das für die zeitliche Änderung der Lichteffizienz der Diode (101) in Abwesenheit eines Lecks in dem abgedichteten Gehäuse repräsentativ ist, und ein Gesetz, das für die zeitliche Änderung der Lichteffizienz der Diode (101) in Anwesenheit eines Lecks in dem abgedichteten Gehäuse repräsentativ ist, und in der Alternative b), ein Gesetz, das für die zeitliche Änderung der Impedanz der Diode (101) in Abwesenheit eines Lecks in dem abgedichteten Gehäuse repräsentativ ist, und ein Gesetz, das für die zeitliche Änderung der Impedanz der Diode (101) in Anwesenheit eines Lecks in dem abgedichteten Gehäuse repräsentativ ist, wobei die Gesetze während einer Charakterisierungsphase beim Design der Vorrichtung bestimmt werden und die Verarbeitungsschaltung in der Lage ist, die Größe mit einem oder mehreren Referenzwerten zu vergleichen, die von den Gesetzen ausgegeben werden, und daraus das mögliche Vorhandensein von Sauerstoff oder Feuchtigkeit mit anormalen Proportionen im Inneren der Hülle abzuleiten,
wobei die Verarbeitungsschaltung (109) in der Lage ist, einen Alarm zu steuern, wenn sie das Vorhandensein von Sauerstoff oder Feuchtigkeit mit anormalen Proportionen innerhalb des Gehäuses detektiert.

2. Vorrichtung nach Anspruch 1, wobei die Messvorrichtung einen Photodetektor (105) aufweist, der gegenüber der organischen lichtemittierenden Diode (101) angeordnet ist.

3. Vorrichtung nach Anspruch 2, wobei der Photodetektor (105) aus einem anorganischen Halbleitermaterial besteht.

4. Medizinische Vorrichtung mit einem abgedichteten biokompatiblen Gehäuse (301) und der Vorrichtung nach einem der Ansprüche 1 bis 3 zum Detektieren eines Lecks in dem abgedichteten Gehäuse (301).

5. Medizinische Vorrichtung nach Anspruch 4, wobei das abgedichtete Gehäuse (301) transparent ist und eine Quelle für die optische Stimulation des Gehirns enthält.

6. Medizinische Vorrichtung nach Anspruch 5, wobei das abgedichtete Gehäuse (301) ein transparentes Rohr (303) aufweist.

7. Medizinische Vorrichtung nach Anspruch 6, wobei das Rohr (303) an seinen Enden durch Kappen (305, 307) hermetisch verschlossen ist.

8. Vorrichtung nach Anspruch 7, aufweisend elektrische Verbindungselemente (313), die wenigstens eine der Kappen (305, 307) durchqueren und Komponenten, die sich im Inneren des Rohrs (303) befinden, elektrisch mit der Außenseite des Gehäuses (301) verbinden.

## Claims

1. A device comprising:
a sealed enclosure;
an organic light-emitting diode (101) placed within the enclosure;
a device (103, 105, 107) for measuring a quantity representative of at least one of the following parameters:
a) the luminous efficiency of the diode (101); and
b) the impedance of the diode (101); and
a processing circuit (109) memorizing, in the alternative a), a law representative of the time variation of the luminous efficiency of the diode (101) in the absence of a leak in the sealed enclosure and a law representative of the time variation of the luminous efficiency of the diode (101) in the presence of a leak in the sealed enclosure, and in the alternative b), a law representative of the time variation of the impedance of the diode (101) in the absence of a leak in the sealed enclosure and a law representative of the time variation of the impedance of the diode (101) in the presence of a leak in the sealed enclosure, said laws being determined during a characterization phase when designing the device, and the processing circuit being capable of comparing said quantity to one or a plurality of reference values output from said laws, and of deducing therefrom the possible presence of oxygen or humidity of abnormal proportions within the enclosure,
wherein the processing circuit (109) is capable of controlling an alarm when it detects a presence of oxygen or of humidity of abnormal proportions within the enclosure.

2. The device of claim 1, wherein the measurement device comprises a photodetector (105) arranged opposite the organic light-emitting diode (101).

3. The device of claim 2, wherein the photodetector (105) is made up of an inorganic semiconductor material.

4. A medical device comprising a sealed biocompatible enclosure (301) and the device of any of claims 1 to 3 for detecting a leak in the sealed enclosure (301).

5. The medical device of claim 4, wherein the sealed enclosure (301) is transparent, and contains a source of optical stimulation of the brain.

6. The medical device of claim 5, wherein the sealed enclosure (301) comprises a transparent tube (303).

7. The medical device of claim 6, wherein the tube (303) is hermetically closed at its ends by caps (305, 307).

8. The device of claim 7, comprising electric connection elements (313) crossing at least one of the caps (305, 307) and electrically coupling to the outside of the enclosure (301) components located inside of the tube (303).
